# EUROPÄISCHE PATENTSCHRIFT

(11) **EP 1 423 359 B1**
(45) Veröffentlichungstag und Bekanntmachung des Hinweises auf die Patenterteilung: **14.07.2010**
(21) Anmeldenummer: 02772164.6
(22) Anmeldetag: 17.08.2002
(51) Int. Cl.: C07C 317/24, C07C 323/22, A61K 31/10, A61P 3/04

(54) **C2-DISUBSTITUIERTE INDAN-1-ONE UND IHRE DERIVATE, VERFAHREN ZU IHRER HERSTELLUNG UND IHRE VERWENDUNG ALS ARZNEIMITTEL**
C2-DISUBSTITUTED INDANE-1-ONES AND THEIR DERIVATIVES, METHOD FOR THEIR PRODUCTION AND THEIR USE AS MEDICAMENTS
INDANE-1-ONES DISUBSTITUES EN C2 ET LEURS DERIVES, LEUR PROCEDE DE PRODUCTION ET LEUR UTILISATION EN TANT QUE MEDICAMENTS

(30) Priorität: 31.08.2001 DE 10142665
(43) Veröffentlichungstag der Anmeldung: 02.06.2004
(73) Patentinhaber: Sanofi-Aventis Deutschland GmbH, 65929 Frankfurt am Main (DE)
(72) Erfinder: JAEHNE, Gerhard, 65929 Frankfurt (DE); KRONE, Volker, 65719 Hofheim (DE); BICKEL, Martin, 61348 Bad Homburg (DE); GOSSEL, Matthias, 65719 Hofheim (DE)
(86) Internationale Anmeldenummer: PCT/EP2002/009204
(87) Internationale Veröffentlichungsnummer: WO 2003/020697

(56) Entgegenhaltungen:
- US-A- 4 129 656
- US-A- 4 174 397
- US-A- 6 090 833
- US-A- 6 159 996
- US-B1- 6 235 763

## Beschreibung

C2-Disubstituierte Indan-1-one und ihre Derivate, Verfahren zu ihrer Herstellung und ihre Verwendung als Arzneimittel

Die Erfindung betrifft C2-disubstituierte Indan-1-one und ihre Derivate sowie deren physiologisch verträgliche Salze und physiologisch funktionelle Derivate.

Bei F. C. Copp et al., J. Chem. Soc. Perkin I, 1983, 909-914, fällt bei der Herstellung von 2-Phenylindan-1-on das 2-Phenyl-2-phenylthioindan-1-on als Zwischenprodukt an.

In WO 97/20806 werden cyclopentyl-substituierte Indan-1-on-Derivate mit u.a. entzündungshemmender Wirkung offenbart.

In WO 98/55439 werden Indan-1-on-Derivate mit zweifacher Substitution am C2 entzündungshemmender Wirkung offenbart.

Der Erfindung lag die Aufgabe zugrunde, Verbindungen zur Verfügung zu stellen, die eine Gewichtsreduktion bei Säugetieren bewirken und die zur Prävention und Behandlung von Obesitas geeignet sind.

Die Erfindung betrifft daher Verbindungen der Formel I, 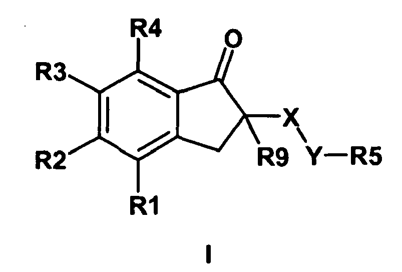 worin bedeuten
- R1, R2, R3, R4: unabhängig voneinander H, F, Cl, Br, CN; N₃, NO₂, OH, O(C₁-C₈)- Alkyl, O(C₃-C₈)-Cycloalkyl, O-CH₂-phenyl, O-phenyl, O-CO-(C₁- C₈)-Alkyl, O-CO-(C₃-C₈)-Cycloalkyl, NH₂, NH-(C₁-C₈)-Alkyl, N[(C₁- C₈)-Alkyl]₂, COOH, CO-O(C₁-C₈)-Alkyl, (C₁-C₈)-Alkyl, (C₃-C₈)- Cycloalkyl, (C₂-C₈)-Alkenyl, (C₂-C₈)-Alkinyl, wobei in den Alkyl-, Alkenyl- und Alkinylgruppen jeweils ein bis zu sieben Wasserstoffatome durch Fluor ersetzt sein können; Phenyl,
1-Imidazolyl;
wobei der Arylrest oder Heterocyclus bis zu zweifach mit F, Cl, Br, CN, OH, (C₁-C₄)-Alkyl, CF₃, O-(C₁-C₄)-Alkyl, substituiert sein kann und in den Alkylgruppen ein bis sieben Wasserstoffatome durch Fluor ersetzt sein können;
- X: S, SO₂;
- Y: (CH₂)ₚ, wobei p gleich 0 oder 1 sein kann;
- R5: CF₃, (C₁-C₈)-Alkyl, wobei in den Alkylgruppen ein bis sieben Wasserstoffatome durch Fluor ersetzt sein können; Phenyl, Pyridinyl, wobei die Ringe bis zu zweifach substituiert sein können mit F, Cl, Br, (C₁-C₈)-Alkyl;
- R9: F, Cl, Br, (C₁-C₈)-Alkyl, wobei in den Alkylgruppen ein bis sieben Wasserstoffatome durch Fluor ersetzt sein können;
(CH₂)ᵤ-Phenyl, wobei Phenyl bis zu zweifach substituiert sein kann mit mit F, Cl, Br, (C₁-C₈)-Alkyl;
wobei die Verbindung der Formel I worin R1, R2, R3 und R4 Wasserstoff, X-Y-R5 S-Phenyl und R9 Phenyl bedeutet, ausgenommen ist,
sowie deren physiologisch verträgliche Salze.

Die Erfindung bezieht sich auf Verbindungen der Formel I, in Form ihrer Racemate, racemischen Mischungen und reinen Enantiomere sowie auf ihre Diastereomere und Mischungen davon.

Die Alkyl-, Alkenyl- und Alkinylreste in den Substituenten R1, R2, R3, R4, R5, R6, R7, R8 und R9 können sowohl geradkettig wie verzweigt sein.

Unter Heterocyclus bzw. Heterocyclischer Rest werden Ringsysteme verstanden, die außer Kohlenstoff noch Heteroatome, wie zum Beispiel Stickstoff, Sauerstoff oder Schwefel enthalten. Ferner gehören auch Ringsysteme zu dieser Definition, worin der Heterocylus bzw. der Heterocyclische Rest mit Benzolkernen kondensiert ist. Bevorzugte Hetercyclen bzw. Heterocyclische Reste sind:
Heteroaryl wie
Benzimidazolyl,
1-[(C₁-C₆)-Alkyl]-Benzimidazolyl,
Imidazolyl,
2- oder 3-Thienyl,
2- oder 3-Furyl,
Benzoxazolyl,
Benzthiazoyl,
2-, 3- oder 4-Pyridyl,
Pyrimidinyl,
4-, 5- oder 6-Pyridazin-2H-3-on-yl,
4-, 5- oder 6-Pyridazin-2-(C₁-C₈)-Alkyl-2H-3-on-yl,
4-, 5- oder 6-Pyridazin-2-benzyl-2H-3-on-yl,
3- oder 4-Pyridazinyl,
2-, 3-, 4- oder 8-Chinolinyl,
1-, 3- oder 4-Isochinolinyl,
1-Phthalazinyl,
3- oder 4-Cinnolinyl,
2- oder 4-Chinazolinyl,
2-Pyrazinyl,
2-Chinoxalinyl,
2-, 4- oder 5-oxazolyl,
3-, 4- oder 5-Isoxazolyl,
2-, 4- oder 5-Thiazolyl,
3-, 4- oder 5-Isothiazolyl,
1-[(C₁-C₆)-Alkyl]-2-, 4- oder 5-Imidazolyl,
3-, 4- oder 5-Pyrazolyl,
1-[(C₁-C₆)-Alkyl]-3-, 4- oder 5-Pyrazolyl,
1- oder 4-[1,2,4]Triazolyl,
4- oder 5-[1,2,3]Triazolyl,
1-[(C₁-C₆)-Alkyl]-4- oder 5-[1.2.3]Triazolyl,
3-, 4- oder 7-Indolyl,
N-[(C₁-C₆)-Alkyl]-3-, 4- oder 7-Indolyl
2-[(C₁-C₆)-Alkyl]-3(2H)-Indazolyl,
1-[(C₁-C₆)-Alkyl]-3(1H)-Indazolyl,
5-Tetrazolyl,
1-[(C₁-C₆)-Alkyl]-1H-Tetrazolyl,
2-[(C₁-C₆)-Alkyl]-2H-Tetrazolyl.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon-, Wein- und Trifluoressigsäure. Für medizinische Zwecke wird in besonders bevorzugter Weise das Chlorsalz verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nichttherapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen. Solche Prodrugs können in vivo zu einer erfindungsgemäßen Verbindung metabolisiert werden. Diese Prodrugs können selbst wirksam sein oder nicht.

Zu den physiologisch funktionellen Derivaten zählen ferner z.B. auch Glucuronide, Schwefelsäureester, Glycoside und Riboside.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze, Solvate und physiologisch funktionellen Derivate wie hierin beschrieben.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht der vom Salz abgeleiteten Verbindungen der Struktur I. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldösis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinalacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wäßrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel (I) mit einem oder mehreren herkömmlichen festen Trägem, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wäßrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Gegenstand der Erfindung ist weiterhin ein Verfahren zur Herstellung der Verbindungen der allgemeinen Formel I, **dadurch gekennzeichnet, daß** man die Verbindungen der Formel I so gewinnt, daß gemäß dem folgenden Reaktionsschema vorgegangen wird:

### Formelschemata

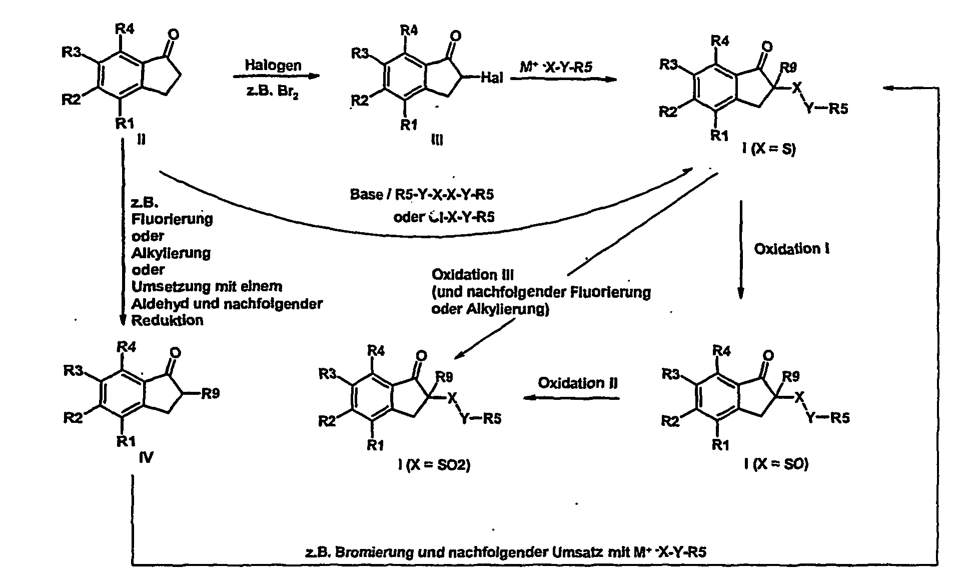

Dazu werden Verbindungen der allgemeinen Formel II, 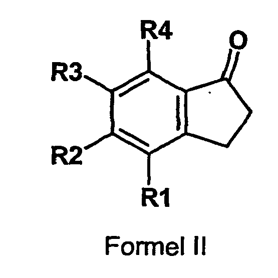 worin R1, R2, R3 und R4 die angegebene Bedeutung besitzen, mit einem Halogen wie z. B. Brom oder Chlor in eine Verbindung der Formel III überführt.

Die Verbindungen der Formel III werden weiter mit Metallsalzen von Thiolen der allgemeinen Formel H-X-Y-R5, wobei X gleich Schwefel ist und Y und R5 die angegebenen Bedeutungen besitzen, in Verbindungen der Formel I mit X = S und R9 = H überführt. Diese Metallsalze können als solche eingesetzt werden oder in Lösung in situ aus dem Thiol und einer Base wie z. B. wässrigem Natriumhydroxid erzeugt werden.

Andererseits kann man Verbindungen der Formel I mit X = S und R9 = H durch Umsatz von Verbindungen der Formel II mit einer Base wie z.B.

Lithiumdiisopropylamid in z.B. Tetrahydrofuran und einem Disulfid der allgemeinen Formel R5-Y-X-X-Y-R5 erhalten, worin R5 und Y die angegebenen Bedeutungen besitzen und X = S ist; alternativ kann statt des Disulfids auch ein Sulfenylchlorid der allgemeinen Formel CI-X-Y-R5, wobei X = S ist und Y und R5 die angegebenen Bedeutungen haben, eingesetzt werden (siehe z.B. D. Seebach et al.; Chem. Ber. 109, 1601-1616 (1976)).

Verbindungen der Formel I, worin X = S und R9 ungleich Wasserstoff ist, lassen sich z.B. folgendermassen gewinnen: Verbindungen der Formel II werden z.B. einer Fluorierung, Alkylierung oder einer Kondensation mit einem Aldehyd und nachfolgender Reduktion unterzogen, so daß man zu Verbindungen der Formel IV gelangt, welche ihrerseits z.B. nach erfolgter Bromierung mit Verbindungen der Formel M^{+ -}X-Y-R5, wobei X = S ist und Y und R5 die oben beschriebenen Bedeutungen haben, zu Verbindungen der Formel I mit X = S und R9 ungleich Wasserstoff umgesetzt werden können.

Verbindungen der Formel I, worin X = SO und R9 ungleich Wasserstoff ist, können z.B. durch selektive Oxidation der Verbindung der Formel I, worin X = S ist, mit einem Äquivalent Peroxytrifluoressigsäure (C. G. Venier et al.; J. Org. Chem. 47, 3773 (1982)) hergestellt werden. Die Herstellung der Sulfoxide aus den Sulfiden kann auch mittels Mangandioxid oder Chromsäure erfolgen (D. Edwards et al.; J. Chem. Soc. 1954, 3272). Für diese Oxidation ist weiterhin auch Wasserstoffperoxid in Essigsäureanhydrid geeignet (A. V. Sviridova et al.; J. Org. Chem (Russ), English Transl.; 7, 2577 (1971).

Verbindungen der Formel I, worin X = SO2 und R9 ungleich Wasserstoff ist, können durch Oxidation mit z.B. 2KHSO₅ x KHSO₄ x K₂SO₄ (Oxone) entweder aus Verbindungen der Formel I, worin X = S und R9 ungleich Wasserstoff ist, oder aus Verbindungen der Formel I, worin X = SO und R9 ungleich Wasserstoff ist, gewonnen werden (siehe z.B. M. Hudlický, Oxidations in Organic Chemistry, ACS Monograph 186, American Chemical Society, Washington, DC, 1990).

Verbindungen der Formel I, worin X = S, SO oder SO2 und R9 ungleich Wasserstoff, z.B. Phenyl, ist, und Y eine Bindung und R5 die beschriebene Bedeutung hat, können auch gewonnen werden, indem Verbindungen der Formel I, worin X = S-Phenyl und R9 = H ist und Y eine Bindung darstellt und R5 die beschriebene Bedeutung hat, z.B. mit Diphenyljodoniumchlorid umgesetzt werden. Die so erhaltenen Verbindungen können entweder schrittweise zu den entsprechenden Verbindungen, worin X = SO oder SO2 ist, umgesetzt werden; oder sie werden nachfolgend mit Zink / Essigsäure entschwefelt und stehen dann für weitere Umsetzungen im Sinne des obigen Schemas zur Verfügung.

Verbindungen der Formel I, worin X = SO oder SO2, R9 = H und Y = eine Bindung (= (CH2)m mit m = 0) ist, können auch alternativ nach dem folgenden Schema (gezeigt für die Darstellung der Arylsulfoxide (H. J. Monteiro et al.; Tetrahedron Letters 11, 921-924 (1975) und Arylsulfone (A. K. Maiti et al.; Tetrahedron 50, 10483-10490 (1994)) hergestellt werden:

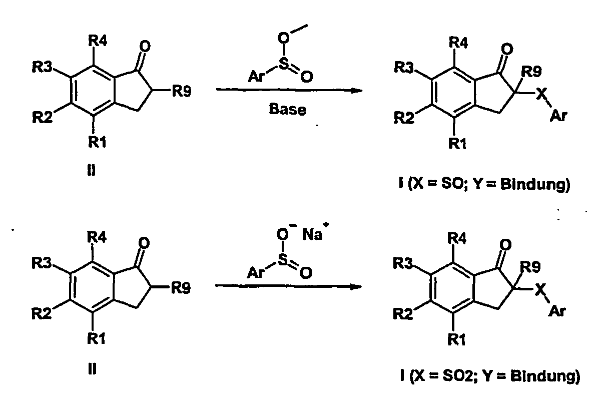

Verbindungen der Formel I, worin X = SO2 und R9 ungleich Wasserstoff ist und Y und R5 die beschriebenen Bedeutungen haben, können auch gewonnen werden, indem Verbindungen der Formel I, worin X = SO2 und R9 = H ist und Y und R5 die beschriebenen Bedeutungen haben, z.B. einer Fluorierung oder Alkylierung unterzogen werden.

Zur Salzbildung kommen als anorganische Säuren beispielsweise in Betracht: Halogenwasserstoffsäuren wie Chlorwasserstoffsäure und Bromwasserstoffsäure, sowie Schwefelsäure, Phosphorsäure und Amidosulfonsäure.

Als organische Säuren zur Salzbildung seien beispielsweise genannt: Ameisensäure, Essigsäure, Benzoesäure, p-Toluolsulfonsäure, Benzolsulfonsäure, Bernsteinsäure, Fumarsäure, Maleinsäure, Milchsäure, Weinsäure, Zitronensäure, L-Ascorbinsäure, Salizylsäure, Isäthionsäure, Methansulfonsäure, Trifluormethansulfonsäure, 1,2-Benzisothiazol-3(2H)-on, 6-Methyl-1,2,3-oxathiazin-4(3H)-on-2,2-dioxid.

Die nachfolgend aufgeführten Beispiele dienen zur Erläuterung der Erfindung, ohne diese jedoch einzuschränken. Die gemessenen Fest-, bzw. Zersetzungspunkte (Fp.) wurden nicht korrigiert und sind generell von der Aufheizgeschwindigkeit abhängig.

Die in untenstehender Tabelle angegebenen Retentionszeiten beziehen sich auf folgende Bestimmungsmethoden:
Methode A: Säule Merck, LiChroCart 55-2, PuroSpher STAR, RP 18 e; Messung bei 254 nm; Gradient: Lösemittel A Acetonitril/Wasser 90:10 + 0.5% Ameisensäure; Lösemittel B Acetonitril/Wasser 10:90 + 0.5 % Ameisensäure; Fluss: 0,750 ml/min; Zeit (min)/Lösemittel B (%): 0.00/95.0, 0.50/95.0, 1.75/5.0, 4.25/5.0, 4.50/95.0, 5.00/95.0; Temperatur: 40°C:
Methode B: Säule: YMC J'sphere, 33x2, ODS H 80 4 µ; Messung bei 254 nm; Gradient: Lösemittel A Acetonitril + 0.5% Ameisensäure; Lösemittel B Wasser + 0.5 % Ameisensäure; Fluss: 1.00 ml/min; Zeit (min)/Lösemittel B (%): 0.00/90.0, 2.50/5.0, 3.30/5.0, 3.35/90.0; Temperatur: 30°C:

**Tabelle 1: Beispiele**

| | | | | | | | | | |
|---|---|---|---|---|---|---|---|---|---|
| 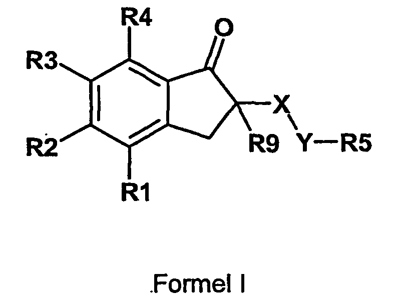 | | | | | | | | | |

| Beispiel | R1 | R2 | R3 | R4 | X | Y | R5 | R9 | Fp. [°C] |
|---|---|---|---|---|---|---|---|---|---|
| 1 | H | Cl | H | H | SO₂ | - | CH3 | F | 150 |
| | | | | | | | | | |
| | | | | | | | | | [MH⁺] |
| 2 | H | Cl | H | H | SO₂ | - | CH₃ | CH₂-Ph | 335,2 |
| 3 | H | OCH₃ | OCH₃ | H | SO₂ | - | CH₃ | F | 289,2 |
| | | | | | | | | | |
| | | | | | | | | | Retentionszeit in Min.(Methode A oder B) |
| 4 | H | Cl | H | H | S | - | pyridin-2-yl | F | 2,789 (A) |
| 5 | H | Cl | H | H | S | CH₂ | CF₃ | F | 2,699 (B) |
| 6 | H | H | H | H | S | - | C₆H₄-4-Cl | F | 3,096 (A) |
| 7 | H | Cl | H | H | SO₂ | - | pyridin-2-yl | F | 1,460 (B) |
| 8 | H | Cl | H | H | SO₂ | CH₂ | CF₃ | F | 1,514 (B) |
| 9 | H | C₆H₄-4-Cl | H | H | SO₂ | - | CH₃ | F | 2,628 (B) |
| 10 | H | CF₃ | H | H | SO₂ | - | CH₃ | F | 2,261 (B) |
| 11 | H | H | C₆H₄-4-CF₃ | H | SO₂ | - | CH₃ | F | 2,656 (B) |
| 12 | Br | H | H | H | SO₂ | - | CH₃ | F | 2,182 (B) |
| 13 | H | N-phthali midoyl | H | H | SO₂ | - | CH₃ | F | 2,221 (B) |

Die Verbindungen der Formel I zeichnen sich durch günstige Wirkungen auf den Fettstoffwechsel aus, insbesondere sind sie zur Gewichtreduktion und nach erfolgter Gewichtsreduktion zum Erhalt eines reduzierten Gewichtes bei Säugetieren und als Anorektika geeignet. Die Verbindungen zeichnen sich durch ihre geringe Toxizität und ihre geringen Nebenwirkungen aus. Die Verbindungen können allein oder in Kombination mit weiteren gewichtsreduzierenden oder anorektischen Wirkstoffen eingesetzt werden. Solche weiteren anorektischen Wirkstoffe werden z.B. in der Roten Liste, Kapitel 01 unter Abmagerungsmittel/Appetitzügler genannt und können auch solche Wirkstoffe beinhalten, die den Energieumsatz des Organismus erhöhen und damit zu einer Gewichtsreduktion führen oder auch solche, welche den allgemeinen Metabolismus des Organismus so beeinflussen, dass eine erhöhte Kalorienzufuhr nicht zu einer Vergrößerung der Fettdepots und eine normale Kalorienzufuhr zu einer Verringerung der Fettdepots des Organismus führt. Die Verbindungen eignen sich zur Prophylaxe sowie insbesondere zur Behandlung von Übergewicht oder Obesitas. Die Verbindungen eignen sich weiterhin zur Prophylaxe sowie insbesondere zur Behandlung von Typ II Diabetes, der Arteriosklerose sowie zur Normalisierung des Lipidstoffwechsels und zur Behandlung des Bluthochdrucks.

Bei einem weiteren Aspekt der Erfindung können die Verbindungen der Formel I in Kombination mit einer oder mehreren weiteren pharmakologisch wirksamen Substanzen verabreicht werden, die beispielsweise ausgewählt sind aus Antidiabetika, Antiadiposita, blutdrucksenkenden Wirkstoffen, Lipidsenkem und Wirkstoffen zur Behandlung und/oder Prävention von Komplikationen, die von Diabetes verursacht werden oder mit Diabetes assoziiert sind.

Geeignete Antidiabetika umfassen Insuline, Amylin, GLP-1- und GLP-2-Derivate wie z.B. diejenigen die in WO 98/08871 von Novo Nordisk A/S offenbart wurden, sowie oral wirksame hypoglykämische Wirkstoffe.

Die oral wirksamen hypoglykämischen Wirkstoffe umfassen vorzugsweise Sulphonylfharnstoffe, Biguanide, Meglitinide, Oxadiazolidindione, Thiazolidindione, Glukosidase-Inhibitoren, Glukagon-Rezeptor-Antagonisten, GLP-1-Agonisten, Kaliumkanalöffner wie z.B. diejenigen, die in WO 97/26265 und WO 99/03861 von Novo Nordisk A/S offenbart wurden, Insulin-Sensitizer, Aktivatoren der Insulin Rezeptor Kinase, Inhibitoren von Leberenzymen, die an der Stimulation der Glukoneogenese und/oder Glykogenolyse beteiligt sind, z.B. Inhibitoren der Glycogenphosphorylase, Modulatoren der Glukoseaufnahme und Glukoseausscheidung, den Fettstoffwechsel verändernde Verbindungen wie antihyperlipidämische Wirkstoffe und antilipidämische Wirkstoffe, z.B. HMGCoA-Reduktase-Inhibitoren, Inhibitoren des Cholesteroltransports/der Cholesterolaufnahme, Inhibitoren der Gallensäurerückresorption oder Inhibitoren des mikrosomalen Triglycerid-Transfer Proteins (MTP), Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die vorliegenden Verbindungen in Kombination mit Insulin verabreicht.

Bei einer weiteren Ausführungsform werden die vorliegenden Verbindungen in Kombination mit einem Sulphonylharnstoff wie z.B. Tolbutamid, Glibenclamid, Glimepirid, Glipizid, Gliquidon, Glisoxepid, Glibornurid oder Gliclazid verabreicht.

Bei einer anderen Ausführungsform werden die vorliegenden Verbindungen in Kombination mit einem Biguanid wie z.B. Metformin verabreicht.

Bei wieder einer anderen Ausführungsform werden die vorliegenden Verbindungen in Kombination mit einem Meglitinid wie z.B. Repaglinid verabreicht.

Bei noch einer weiteren Ausführungsform werden die vorliegenden Verbindungen in Kombination mit einem Thiazolidindion wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei noch einer weiteren Ausführungsform werden die vorliegenden Verbindungen in Kombination mit einem Monoamine Oxidase Inhibitor, wie z.B. in WO 01/12176 offenbart, verabreicht. Besonders geeignet sind dabei [3(S),3a(S)]-3-methoxymethyl-7-[4,4,4-trifluorobutoxy]-3,3a,4,5-tetrahydro-1H-oxazolo[3,4-a]quinolin-1-on, (R)-5-(methoxymethyl)-3-[6-(4,4,4-trifluorobutoxy)benzofuran-3-yl]oxazolidin-2-one oder (R)-5-(methoxymethyl)-3-[6-cyclopropylmethoxybenzofuran-3-yl]oxazolidin-2-one.

Bei einer weiteren Ausführungsform werden die vorliegenden Verbindungen in Kombination mit einem α-Glukosidase-Inhibitor wie z.B. Miglitol oder Acarbose verabreicht.

Bei noch einer weiteren Ausführungsform werden die vorliegenden Verbindungen in Kombination mit einem hCNTF (human ciliary neurotrophic factor) oder Derivaten davon wie z.B. CNTF_{AX15} oder modifiziertes CNTF_{AX15}, wie z.B. in Lambert et al., PNAS 98, 4652-4657 offenbart, verabreicht.

Bei einer anderen Ausführungsform werden die vorliegenden Verbindungen in Kombination mit einem Wirkstoff verabreicht, der auf den ATP-abhängigen Kaliumkanal der Betazellen wirkt, wie z.B. Tolbutamid, Glibenclamid, Glimepirid, Glipizid, Gliclazid oder Repaglinid.

Bei noch einer anderen Ausführungsform werden die vorliegenden Verbindungen in Kombination mit einem antihyperlipidämischen Wirkstoff oder einem antilipidämischen Wirkstoff wie z.B. Cholestyramin, Colestipol, Clofibrat, Gemfibrozil, Lovastatin, Pravastatin, Simvastatin, Atorvastatin, Cerivastatin, Fluvastatin, Probucol, Ezetimibe oder Dextrothyroxin verabreicht.

Bei einer weiteren Ausführungsform werden die vorliegenden Verbindungen in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht. Weiterhin können die erfindungsgemäßen Verbindungen in Kombination mit einem oder mehreren Antiadiposita oder appetitregulierenden Wirkstoffen verabreicht werden.

Solche Wirkstoffe können ausgewählt werden aus der Gruppe bestehend aus CART-Agonisten, NPY-Antagonisten, Melanocortin 3 oder 4 (MC3 oder MC4) - Agonisten, Melanin-konzentrierendes Hormon (MCH) - Antagonisten, Orexin-Antagonisten, H3-Agonisten, TNF-Agonisten, CRF-Agonisten, CRF BP-Antagonisten, Urocortin-Agonisten, β3-AdrenoceptorAgonisten, CCK-Agonisten, Serotonin-Wiederaufnahme-Inhibitoren, gemischte Sertonin- und Noradrenalin-Wiederaufnahme-Inhibitoren, 5HT-Modulatoren, Bombesin-Agonisten, Galanin-Antagonisten, Glucocorticoid Rezeptor Modulatoren, Wachstumshormon, Wachstumshormon freisetzende Verbindungen, TRH-Agonisten, Modulatoren der Entkopplungsproteine 2 oder 3, Leptin Rezeptor Agonisten, Leptinmimetika, Dopamin-Agonisten (Bromocriptin, Doprexin), Lipase/Amylase-Inhibitoren, Antagonisten des Cannabinoid Rezeptors 1, Modulatoren des die Acylierung stimulierendes Protein (ASP), PPAR-Modulatoren, RXR-Modulatoren oder TR-β-Agonisten.

Bei einer Ausführungsform der Erfindung ist das Antiadipositum Leptin oder modifiziertes Leptin.

Bei einer anderen Ausführungsform ist das Antiadipositum Dexamphetamin oder Amphetamin.

Bei einer anderen Ausführungsform ist das Antiadipositum Fenfluramin oder Dexfenfluramin.

Bei noch einer anderen Ausführungsform ist das Antiadipositum Sibutramin oder die mono- und bisdemethylierten Wirkmetabolite von Sibutramin.

Bei einer weiteren Ausführungsform ist das Antiadipositum Orlistat.

Bei einer anderen Ausführungsform ist das Antiadipositum Mazindol, Diethylpropion oder Phentermin.

Weiterhin können die vorliegenden Verbindungen in Kombination mit einem oder mehreren antihypertensiven Wirkstoffen verabreicht werden. Beispiele für antihypertensive Wirkstoffe sind Betablocker wie Alprenolol, Atenol, Timolol, Pindolol, Propanolol und Metoprolol, ACE (Angiotensin Converting Enzym)-Hemmer wie z.B. Benazepril, Captopril, Enalapril, Fosinopril, Lisinopril, Quinapril und Rampril, Calciumkanal-Blocker wie Nifedipin, Felodipin, Nicardipin, Isradipin, Nimodipin, Diltiazem und Verapamil, sowie Alphablocker wie Doxazosin, Urapidil, Prazosin und Terazosin. Weiterhin kann verwiesen werden auf Remington: The Science and Practice of Pharmacy, 19. Auflage, Gennaro, Hrsg., Mack Publishing Co., Easton, PA, 1995.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

Die Wirksamkeit der Verbindungen der Formel I wurde wie folgt getestet:

### Biologisches Prüfmodell:

Die Prüfung der anorektischen Wirkung erfolgte an weiblichen NMRI Mäusen. Nach 24stündigem Futterentzug wurde das Testpräparat intraperitoneal (ip) oder über eine Schlundsonde (po) verabreicht. In Einzelhaltung und bei freiem Zugang zu Trinkwasser wurde den Tieren 30 Minuten nach Präparatgabe Kondensmilch angeboten. Der Kondensmilchverbrauch wurde halbstündlich 7 Stunden lang bestimmt und das Allgemeinbefinden der Tiere beobachtet. Der gemessene Milchverbrauch wurde mit dem unbehandelter Kontrolltieren verglichen.

**Tabelle 2: Anorektische Wirkung, gemessen als Reduktion des kumulierten Milchkonsums behandelter im Vergleich zu dem unbehandelter Tiere.**

| Verbindung/Beispiel 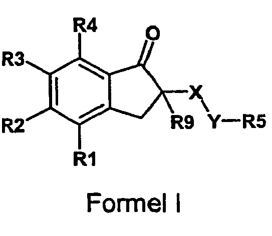 | Dosis [mg/kg] | Anzahl der Tiere / Kumulierter Milchkonsum der behandelten Tiere N / [ml] | Anzahl der Tiere / Kumulierter Milchkonsum der unbehandelten Kontrolltiere N / [ml] | Reduktion des kumulierten Milchkonsums in % der Kontrolle |
|---|---|---|---|---|
| | | | | |
| Beispiel 1 | 20 | 5 / 2.00 | 5 / 3.86 | 48 |

Aus der Tabelle ist abzulesen, daß die Verbindungen der Formel I eine sehr gute anorektische Wirkung zeigen.

Nachfolgend wird die Herstellung einiger Beispiele detailliert beschrieben, die übrigen Verbindungen der Formel I wurden analog erhalten:

### Beispiel 1: 5-Chlor-2-fluor-2-methansulfonyl-indan-1-on:

1. 5-Chlor-2-methylsulfanyl-indan-1-on:
   0.98 g (4 mmol) 2-Brom-5-chlor-indan-1-on und 0.42 g (6 mmol) Natriumthiomethylat werden in 5 ml Ethanol suspendiert, 30 Minuten im Ultraschallbad behandelt und anschließend 90 Minuten bei Raumtemperatur gerührt. Die Reaktionsmischung wird im Vakuum eingeengt und über Kieselgel mit Toluol/Essigsäureethylester 10/1 chromatographiert. Die Eluate werden im Vakuum eingeengt und liefern 0.63 g 5-Chlor-2-methylsulfanyl-indan-1-on mit dem Schmelzpunkt 90°C.
2. 5-Chlor-2-methansulfonyl-indan-1-on:
   0.5 g (2.35 mmol) 5-Chlor-2-methylsulfanyl-indan-1-on werden in 10 ml Methanol gelöst; bei 0°C wird eine Lösung aus 4.33 g (7.05 mmol) 2KHSO₅ x KHSO₄ x K₂SO₄ in 10 ml Wasser zugetropft. Die Mischung wird 5 h bei Raumtemperatur gerührt; das Methanol wird abdestilliert und der wässrige Rückstand wird mit Dichlormethan extrahiert. Die organische Phase wird abgetrennt, über MgSO₄ getrocknet, filtriert und im Vakuum eingeengt. Man erhält 0.5 g 5-Chlor-2-methansulfonyl-indan-1-on mit dem Schmelzpunkt 197°C.
3. 5-Chlor-2-fluor-2-methansulfonyl-indan-1-on:
   0.734 g (3 mmol) 5-Chlor-2-methansulfonyl-indan-1-on und 1.77 g (5 mmol) N-Fluor-N'-(chlormethyl)triethylendiamin bis(tetrafluorborat) werden in einer Mischung aus 2.5 ml Wasser und 7.5 ml Acetonitril suspendiert und 4 h unter Rückfluß gerührt. Die Reaktionsmischung wird abgekühlt, im Vakuum eingeengt und über Kieselgel mit Dichlormethan als Laufmittel chromatographisch gereinigt. Man erhält 5-Chlor-2-fluor-2-methansulfonyl-indan-1-on mit dem Schmelzpunkt 150°C.

### Beispiel 2: 2-Benzyl-5-chlor-2-methansulfonyl-indan-1-on:

1. 2-Benzyl-5-chlor-1-oxo-indan-2-carbonsäure methylester:
   1,11 ml Diisopropylamin werden in 25 ml trockenem Tetrahydrofuran gelöst, bei -50°C mit 6,9 ml N-Butyllithium in n-Hexan (15%ig) versetzt, und 20 Minuten bei -10°C gerührt. Danach werden bei -50°C 2,25 g 5-Chlor-1-oxo-indan-2-carbonsäuremethylester, gelöst in 25 ml Tetrahydrofuran, zugetropft. Die Mischung wird 30 min bei -50°C gerührt und dann mit einer Lösung von 1,31 ml Benzylbromid in 5 ml Tetrahydrofuran tropfenweise versetzt. Das Reaktionsgemisch wird 20 h bei Raumtemperatur gerührt. Zur Vervollständigung der Reaktion werden nochmals 1,31 ml Benzylbromid nachgegeben und die Mischung 72 h unter Rückfluss erwärmt. Die Reaktionsmischung wird mit 20 ml gesättigter Natriumhydrogencarbonatlösung versetzt und mit Essigsäureethylester verdünnt. Die organische Phase wird abgetrennt, mit gesättigter Kochsalzlösung gewaschen, über Magnesiumsulfat getrocknet, filtriert und im Vakuum eingeengt. Man erhält 2-Benzyl-5-chlor-1-oxo-indan-2-carbonsäure methylester mit dem Molekulargewicht 314 (C₁₈H₁₅ClO₃); MS (ESI): 315,1 (MH⁺).
2. 2-Benzyl-5-chlor-indan-1-on:
   Die Verbindung des Beispiels 2.1. wird in einer Mischung aus 10 ml Ethanol mit 10 ml 5%iger Natronlauge suspendiert und eine Stunde bei 40°C gerührt. Danach wird der Alkohol im Vakuum entfernt, und der Rückstand wird mit Essigsäureethylester extrahiert. Die organische Phase wird mit Wasser neutral gewaschen, über Magnesiumsulfat getrocknet, filriert und im Vakuum eingeengt. Der Rückstand wird über Kieselgel mit n-Heptan/Essigsäureethylester2/1 chromatographische gereinigt. Man erhält 2-Benzyl-5-chlor-indan-1-on mit dem Molekulargewicht 256 (C₁₆H₁₃ClO); MS (ESI): 256,8 (MH⁺).
3. 2-Benzyl-2-brom-5-chlor-indan-1-on:
   0,86 g der Verbindung des Beispiels 2.2 werden in 4 ml Essigsäure gelöst, mit 50 µl 48%iger HBr-Lösung und 0,207 ml Brom versetzt und eine Stunde bei Raumtemperatur gerührt. Das Rohprodukt wird über Kieselgel mit Toluol chromatographisch gereinigt. Man erhält 2-Benzyl-2-brom-5-chlor-indan-1-on mit dem Molekulargewicht 334 (C₁₆H₁₂BrClO); MS (ESI): 334,8 (MH⁺).
4. 2-Benzyl-5-chlor-2-methansulfonyl-indan-1-on:
   1 mmol der Verbindung des Beispiels 2.3 werden mit 1,2 mmol Methansulfinsäurenatriumsalz in 3 ml Dimethylformamid 4 Stunden bei 70°C gerührt. Das Rohprodukt wird über Kieselgel mit n-Heptan/Essigsäureethylester 1/1 chromatographisch gereinigt. Man erhält 2-Benzyl-5-chlor-2-methansulfonyl-indan-1-on mit dem Molekulargewicht 334 (C₁₇H₁₅ClO₃S); MS (ESI): 335,2 (MH⁺).

Die Verbindungen der Beispiele 4 - 6 werden durch Umsatz des entsprechenden 2-Fluorindan-1-ons mit dem Natriumsalz des entsprechenden Mercaptans gewonnen.

Die Verbindungen der Beispiele 7 - 13 werden wie in Beispiel 1.3 beschrieben durch Fluorierung der entsprechenden Sulfonylderivate erhalten.

## Patentansprüche

1. Verbindungen der Formel I, 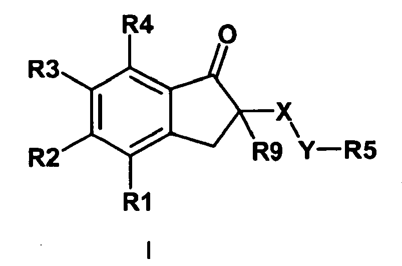 worin bedeuten
R1, R2, R3, R4 unabhängig voneinander H, F, Cl, Br, CN; N₃, NO₂, OH, O(C₁-C₈)- Alkyl, O(C₃-C₈)-Cycloalkyl, O-CH₂-phenyl, O-phenyl, O-CO-(C₁- C₈)-Alkyl, O-CO-(C₃-C₈)-Cycloalkyl, NH₂, NH-(C₁-C₈)-Alkyl, N[(C₁- C₈)-Alkyl]₂, COOH, CO-O(C₁-C₈)-Alkyl, (C₁-C₈)-Alkyl, (C₃-C₈)- Cycloalkyl, (C₂-C₈)-Alkenyl, (C₂-C₈)-Alkinyl, wobei in den Alkyl-, Alkenyl- und Alkinylgruppen jeweils ein bis zu sieben Wasserstoffatome durch Fluor ersetzt sein können; Phenyl,
1-Imidazolyl;
wobei der Arylrest oder Heterocyclus bis zu zweifach mit F, Cl, Br, CN, OH, (C₁-C₄)-Alkyl, CF₃, O-(C₁-C₄)-Alkyl, substituiert sein kann und in den Alkylgruppen ein bis sieben Wasserstoffatome durch Fluor ersetzt sein können;
X S, SO₂;
Y (CH₂)ₚ, wobei p gleich 0 oder 1 sein kann;
R5 CF₃, (C₁-C₈)-Alkyl, wobei in den Alkylgruppen ein bis sieben Wasserstoffatome durch Fluor ersetzt sein können; Phenyl, Pyridinyl, wobei die Ringe bis zu zweifach substituiert sein können mit F, Cl, Br, (C₁-C₈)-Alkyl;
R9 F, Cl, Br, (C₁-C₈)-Alkyl, wobei in den Alkylgruppen ein bis sieben Wasserstoffatome durch Fluor ersetzt sein können; (CH₂)ᵤ-Phenyl, wobei Phenyl bis zu zweifach substituiert sein kann mit F, Cl, Br, (C₁-C₈)-Alkyl;
wobei die Verbindung der Formel I worin R1, R2, R3 und R4 Wasserstoff, X-Y-R5 S-Phenyl und R9 Phenyl bedeutet, ausgenommen ist,
sowie deren physiologisch verträgliche Salze.

2. Arzneimittel enthaltend eine oder mehrere der Verbindungen gemäß Anspruch 1.

3. Arzneimittel enthaltend eine oder mehrere der Verbindungen Anspruch 1 und ein oder mehrere Wirkstoffe zur Gewichtreduktion bei Säugetieren.

4. Verbindungen gemäß Anspruch 1 zur Anwendung als Medikament zur Gewichtsreduktion bei Säugetieren.

5. Verbindungen gemäß Anspruch 1 zur Anwendung als Medikament zur Prophylaxe oder Behandlung der Obesitas.

6. Verbindungen gemäß Anspruch 1 zur Anwendung als Medikament zur Prophylaxe oder Behandlung des Typ II Diabetes.

7. Verbindungen gemäß Anspruch 1 in Kombination mit mindestens einem weiteren zur Gewichtreduktion bei Säugetieren geeigneten Wirkstoff zur Anwendung als Medikament zur Prophylaxe oder Behandlung der Obesitas.

8. Verbindungen gemäß Anspruch 1 in Kombination mit mindestens einem weiteren zur Gewichtreduktion bei Säugetieren geeigneten Wirkstoff zur Anwendung als Medikament zur Prophylaxe oder Behandlung der des Typ II Diabetes.

9. Verwendung der Verbindungen gemäß Anspruch 1 zur Herstellung eines Medikaments zur Prophylaxe oder Behandlung der Obesitas.

10. Verwendung der Verbindungen gemäß Anspruch 1 zur Herstellung eines Medikaments zur Prophylaxe oder Behandlung des Typ II Diabetes.

## Claims

1. A compound of the formula I, 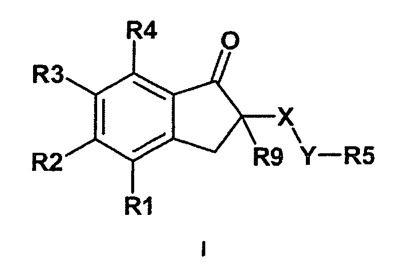 in which
R1, R2, R3, R4 independently of one another are H, F, Cl, Br, CN; N₃, NO₂, OH, O(C₁-C₈)-alkyl, O(C₃-C₈)-cycloalkyl, O-CH₂-phenyl, O-phenyl, O-CO-(C₁-C₈)-alkyl, O-CO- (C₃- C₈)-cycloalkyl, NH₂, NH-(C₁-C₈)-alkyl, N[(C₁-C₈)-alkyl]₂, COOH, CO-O(C₁-C₈)- alkyl, (C₁-C₈)-alkyl, (C₃-C₈)-cycloalkyl, (C₂-C₈)-alkenyl, (C₂-C₈)-alkynyl, where in the alkyl, alkenyl and alkynyl groups in each case one to seven hydrogen atoms may be replaced by fluorine; phenyl,
1-imidazolyl;
where the aryl radical or heterocycle may be substituted up to two times by F, Cl, Br, CN, OH, (C₁-C₄)-alkyl, CF₃, O-(C₁-C₄)-alkyl, and where in the alkyl groups one to seven hydrogen atoms may be replaced by fluorine;
X is S, SO₂;
Y is (CH₂)ₚ, where p may be 0 or 1;
R5 is CF₃, (C₁-C₈)-alkyl, where in the alkyl groups one to seven hydrogen atoms may be replaced by fluorine; phenyl, pyridinyl, where the rings may be substituted up to two times by F, Cl, Br, (C₁- C₈)-alkyl;
R9 is F, Cl, Br, (C₁-C₈)-alkyl, where in the alkyl groups one to seven hydrogen atoms may be replaced by fluorine; (CH₂)ᵤ-phenyl, where phenyl may be substituted up to two times by F, Cl, Br, (C₁-C₈)-alkyl;
except for the compound of the formula I in which R1, R2, R3 and R4 are hydrogen, X-Y-R5 is S-phenyl and R9 is phenyl,
and its physiologically acceptable salts.

2. A pharmaceutical comprising one or more compounds as claimed in claim 1.

3. A pharmaceutical comprising one or more compounds as claimed in claim 1 and one or more active compounds for reducing weight in mammals.

4. A compound as claimed in claim 1 for use as a medicament for reducing weight in mammals.

5. A compound as claimed in claim 1 for use as a medicament for the prophylaxis or treatment of obesity.

6. A compound as claimed in claim 1 for use as a medicament for the prophylaxis or treatment of type II diabetes.

7. A compound as claimed in claim 1 in combination with at least one further active compound suitable for reducing weight in mammals for use as a medicament for the prophylaxis or treatment of obesity.

8. A compound as claimed in claim 1 in combination with at least one further active compound suitable for reducing weight in mammals for use as a medicament for the prophylaxis or treatment of type II diabetes.

9. The use of a compound as claimed in claim 1 for preparing a medicament for the prophylaxis or treatment of obesity.

10. The use of a compound as claimed in claim 1 for preparing a medicament for the prophylaxis or treatment of type II diabetes.

## Revendications

1. Composés de formule I, 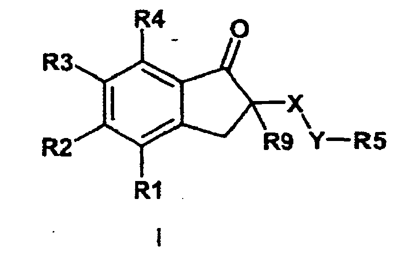 où
R1, R2, R3, R4 signifient, indépendamment l'un de l'autre H, F, Cl, Br, CN ; N₃, NO₂, OH, O(C₁-C₈)- alkyle, O(C₃-C₈)-cycloalkyle, O-CH₂-phényle, O- phényle, O-CO-(C₁-C₈)-alkyle, O-CO-(C₃-C₈)- cycloalkyle, NH₂, NH-(C₁-C₈)-alkyle, N[(C₁-C₈)- alkyle]₂, COOH, CO-O(C₁-C₈)-alkyle, (C₁-C₈)-alkyle, (C₃-C₈)-cycloalkyle, (C₂-C₈)-alcényle, (C₂-C₈)- alcynyle, où, dans les groupes alkyle, alcényle et alcynyle, à chaque fois un à sept atomes d'hydrogène peuvent être remplacés par fluor ;
phényle,
1-imidazolyle ; où le radical aryle ou l'hétérocycle peut être jusqu'à disubstitué par F, Cl, Br, CN, OH, (C₁-C₄)- alkyle, CF₃, O-(C₁-C₄)-alkyle et, dans les groupes alkyle, un à sept atomes d'hydrogène peuvent être remplacés par fluor ;
X signifie S, SO₂ ;
Y signifie (CH₂)ₚ, où p peut valoir 0 ou 1 ;
R5 signifie CF₃, (C₁-C₈)-alkyle, où, dans les groupes alkyle, un à sept atomes d'hydrogène peuvent être remplacés par fluor ;
phényle, pyridinyle, où les cycles peuvent être jusqu'à disubstitués par F, Cl, Br, (C₁-C₈)- alkyle ;
R9 signifie F, Cl, Br, (C₁-C₈)-alkyle, où, dans les groupes alkyle, un à sept atomes d'hydrogène peuvent être remplacés par fluor ;
(CH₂)ᵤ-phényle, où phényle peut être jusqu'à disubstitué par F, Cl, Br, (C₁-C₈)-alkyle ;
où le composé de formule I, dans laquelle R1, R2, R3 et R4 signifient hydrogène, X-Y-R5 signifie S-phényle et R9 signifie phényle, est exclu,
ainsi que leurs sels physiologiquement acceptables.

2. Médicament contenant un ou plusieurs des composés selon la revendication 1.

3. Médicament contenant un ou plusieurs des composés selon la revendication 1 et une ou plusieurs substances actives pour la réduction pondérale chez les mammifères.

4. Composés selon la revendication 1 pour une utilisation comme médicament pour la réduction pondérale chez les mammifères.

5. Composés selon la revendication 1 pour une utilisation comme médicament destiné à la prophylaxie ou au traitement de l'obésité.

6. Composés selon la revendication 1 pour une utilisation comme médicament destiné à la prophylaxie ou au traitement du diabète du type II.

7. Composés selon la revendication 1 en combinaison avec au moins une autre substance active appropriée pour la réduction pondérale chez les mammifères pour l'utilisation comme médicament destiné à la prophylaxie ou au traitement de l'obésité.

8. Composés selon la revendication 1 en combinaison avec au moins une autre substance active appropriée pour la réduction pondérale chez les mammifères pour l'utilisation comme médicament destiné à la prophylaxie ou au traitement du diabète de type II.

9. Utilisation des composés selon la revendication 1 pour la préparation d'un médicament destiné à la prophylaxie ou au traitement de l'obésité.

10. Utilisation des composés selon la revendication 1 pour la préparation d'un médicament destiné à la prophylaxie ou au traitement du diabète de type II.
